(19) Europäisches Patentamt
European Patent Office
Office européen des brevets

(11) **EP 3 870 035 B1**

(12) # EUROPÄISCHE PATENTSCHRIFT

(45) Veröffentlichungstag und Bekanntmachung des Hinweises auf die Patenterteilung:
**27.03.2024 Patentblatt 2024/13**

(21) Anmeldenummer: **19804629.4**

(22) Anmeldetag: **21.10.2019**

(51) Internationale Patentklassifikation (IPC):
**A61B 5/028** (2006.01)   **A61B 5/029** (2006.01)
**A61M 1/36** (2006.01)

(52) Gemeinsame Patentklassifikation (CPC):
**A61B 5/028; A61B 5/029; A61M 1/3609; A61M 1/361; A61M 1/3612;** A61M 2205/3368

(86) Internationale Anmeldenummer:
**PCT/EP2019/078571**

(87) Internationale Veröffentlichungsnummer:
**WO 2020/083836 (30.04.2020 Gazette 2020/18)**

(54) **SYSTEM, COMPUTERSYSTEM UND COMPUTERPROGRAMM ZUR BESTIMMUNG EINES KARDIOVASKULÄREN PARAMETERS**

SYSTEM, COMPUTER SYSTEM AND COMPUTER PROGRAM FOR DETERMINING A CARDIOVASCULAR PARAMETER

PROCÉDÉ, SYSTÈME INFORMATIQUE ET PROGRAMME INFORMATIQUE POUR LA DÉTERMINATION D'UN PARAMÈTRE CARDIOVASCULAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.10.2018 DE 102018008356**

(43) Veröffentlichungstag der Anmeldung:
**01.09.2021 Patentblatt 2021/35**

(73) Patentinhaber: **Pulsion Medical Systems SE 85622 Feldkirchen (DE)**

(72) Erfinder:
- **HUBER, Wolfgang**
  **83703 Gmund (DE)**
- **KONRAD, Mark**
  **81541 München (DE)**
- **KAMMERZELL, Sergej**
  **81827 München (DE)**

(74) Vertreter: **Kehl, Ascherl, Liebhoff & Ettmayr Patentanwälte Partnerschaft mbB Emil-Riedel-Straße 18 80538 München (DE)**

(56) Entgegenhaltungen:
**US-A1- 2011 004 141**

- **DATABASE MEDLINE [Online] US NATIONAL LIBRARY OF MEDICINE (NLM), BETHESDA, MD, US; April 2013 (2013-04), ZERAATI ABBASALI ET AL: "A review article: access recirculation among end stage renal disease patients undergoing maintenance hemodialysis.", Database accession no. NLM23841034 & ZERAATI ABBASALI ET AL: "A review article: access recirculation among end stage renal disease patients undergoing maintenance hemodialysis.", NEPHRO-UROLOGY MONTHLY SPRING 2013, vol. 5, no. 2, April 2013 (2013-04), pages 728-732, ISSN: 2251-7006**

## Beschreibung

## Technisches Gebiet

Die vorliegende Erfindung betrifft ein System zur Bestimmung eines oder mehrerer kardiovaskulärer Parameter eines Patienten, welches dazu eingerichtet ist, mit einer Vorrichtung zur extrakorporalen Blutbehandlung zusammenzuwirken.

## Stand der Technik

Eine extrakorporale Blutbehandlung ist für eine Reihe von Krankheitsbildern in Patienten indiziert, beispielsweise die Hämodialyse bei Nieren- und Lebererkrankungen sowie die extrakorporale Decarboxylierung / Membranoxygenierung (ECMO) bei der Therapie schwerer Lungenerkrankungen (akutes Atemnotsyndrom, ARDS) und von Herzerkrankungen (Myokardinfarkt, schwere Rhythmusstörungen). Die extrakorporale Membranoxygenierung gehört dabei zur Standardtherapie für die Stabilisierung des Blutkreislaufs und/oder der Atemfunktion von Patienten mit ARDS und lebensbedrohlicher Hypoxämie. Bei der ECMO werden großvolumige Venen mittels Seldinger-Technik kanüliert, sodass ein Blutfluss zwischen Patient und der ECMO ermöglicht wird. In der ECMO-Vorrichtung wird das Blut mittels einer Pumpe über einen Membranoxygenator perfundiert. Dieser beherbergt eine semipermeable Membran, über welche der Gasaustausch stattfindet, indem auf einer Seite das Patientenblut fließt und auf der anderen Seite Sauerstoff eingeleitet wird. Die Pumpe produziert dabei einen Blutfluss von 2 bis 6 l/min, sodass eine effiziente Oxygenierung und Kohlendioxidelimination möglich ist.

Am häufigsten erfolgt der Zugang zum Gefäßsystem des Patienten über die Femoralgefäße. In Abhängigkeit von der zugrunde liegenden Erkrankung des Patienten sind zur Etablierung der ECMO eine venovenöse, eine arterio-venöse und eine veno-arterielle Kanülierung möglich. Die arterio-venöse Kanülierung wird bei Patienten mit ausschließlich gestörtem Gasaustausch, also bei stabilem Kreislauf und guter Herzfunktion, eingesetzt (avECMO; bei intakter Herzfunktion z.T. Betrieb ohne Pumpe möglich). Dabei wird der Blutfluss von der A. femoralis über den Oxygenator zurück in den rechten Vorhof geleitet. Dem gegenüber steht die veno-arterielle ECMO (vaECMO), die eine komplette Herzunterstützung ermöglicht. Die venöse Drainage erfolgt über die V. cava inferior, von dort gelangt das Blut in den Oxygenator und über die A. femoralis oder die Aorta ascendens wieder zum Patienten. Über die Drainage des rechten Vorhofes kann mit der vaECMO eine biventrikuläre Entlastung erreicht und zusätzlich ein begleitendes Lungenversagen therapiert werden. Am häufigsten wird die veno-venöse Kanülierung eingesetzt; die wECMO dient als Lungenunterstützung dem Gasaustausch der Lunge. Das Blut des Patienten fließt über eine in der V. cava inferior liegende Kanüle zum Oxygenator und von dort über die V. cava superior zurück in den Patientenkreislauf.

Obwohl das Verfahren seit vielen Jahrzehnten verwendet wird, konnten prospektivrandomisierte Studien bei ARDS Patienten, bei denen der Einsatz von ECMO im Vergleich zur konventionellen Behandlung überprüft wurde, bislang nur eine marginale Verbesserung des Outcomes mit strittigem Überlebensvorteil durch die ECMO aufzeigen. Strategien zur Verbesserung der Wirksamkeit der ECMO umfassen optimierte Patientenauswahl, weniger invasive Techniken und Kombination von ECMO mit ultra-protektiver Beatmung. Außerdem wird postuliert, dass die Optimierung der hämodynamischen Situation des Patienten vor der eigentlichen ECMO dazu beitragen kann, Patienten besser in die ECMO zu integrieren und die ECMO-Konfiguration optimal anzupassen. Dabei ist der extrakorporale Blutfluss in der ECMO, der an kardiovaskuläre Parameter wie z. B. das Herzzeitvolumen (HZV) des Patienten angepasst wird, von großer Bedeutung für eine effektive Sauerstoffversorgung des Blutes. Die Optimierung der hämodynamischen Situation des Patienten erfordert eine genaue Messung kardiovaskulärer Parameter vor und während ECMO, beispielsweise mittels Indikatorverdünnungstechnik wie Thermodilution. Pulmonal-arterielle und transpulmonale Thermodilutionsmessungen während der ECMO-Behandlung können allerdings zu einer Fehleinschätzung kardiovaskulärer Parameter führen, da der extrakorporale Kreislauf einen Indikatorverlust im Gefäßsystem des Patienten bedingen kann. Beispielsweise kann das aus derartigen Messungen berechnete Herzzeitvolumen über dem tatsächlichen Herzzeitvolumen liegen. Es ist daher die Aufgabe der vorliegenden Erfindung, den Fehler bei der Bestimmung kardiovaskulärer Parameter, wie z.B. des HZV, während einer Behandlung mit einer extrakorporalen Blutbehandlungsvorrichtung, z. B. während einer ECMO Behandlung, zu erfassen, zu minimieren und möglichst weitgehend zu korrigieren. Durch das erfindungsgemäße System werden Genauigkeit und Zuverlässigkeit der Bestimmung kardiovaskulärer Parameter, wie z.B. des HZV, während einer Behandlung mit einer extrakorporalen Blutbehandlungsvorrichtung erhöht; somit werden die Patienten besser in die entsprechende extrakorporale Blutbehandlungsvorrichtung integriert und deren Konfiguration kann optimal angepasst werden.

US 2011/004143 A1 offenbart ein Verfahren und eine Anordnung zur Bestimmung der Rezirkulation in einer Fistel und/oder des kardio-pulmonalen Anteils der Rezirkulation während der extrakorporalen Behandlung von Blut bei einem Patienten, wie z. B. in der Hämodialyse. Ein Temperatursensor in der venösen Blutleitung erfasst dabei unmittelbar eine auf der Blutseite des Dialysators induzierte Temperaturänderung.

## Beschreibung der Erfindung

Die der Erfindung zugrunde liegende Aufgabe wird durch ein System gemäß Anspruch 1 gelöst. Besondere Ausführungsformen können gemäß einem der Ansprüche 2 bis 10 gestaltet sein.

In einem ersten Aspekt betrifft die Erfindung daher ein System zum Bestimmen eines kardiovaskulären Parameters eines Patienten. Das System kann dabei auch vorteilhaft dazu ausgelegt sein, mehrere kardiovaskuläre Parameter eines Patienten zu bestimmen. Das System ist dazu eingerichtet, mit einer Vorrichtung zur extrakorporalen Blutbehandlung zusammenzuwirken, welche mit dem venösen Gefäßsystem des Patienten über einen zuführenden und einen abführenden Schenkel verbunden ist. Bei der extrakorporalen Blutbehandlungsvorrichtung kann es sich beispielsweise um eine Vorrichtung zur Hämodialyse, eine Vorrichtung zur Leberunterstützung, oder um eine extrakorporale Vorrichtung zur Decarboxylierung oder zur Membranoxygenierung handeln. Der zuführende Schenkel führt der Blutbehandlungsvorrichtung Blut aus dem Gefäßsystem des Patienten zu. Beispielsweise leitet bei der vvECMO ein in der unteren Hohlvene liegender "Drainage"-Katheter venöses Blut zum Membranoxygenator. Das behandelte Blut wird über den abführenden Schenkel wieder in das Gefäßsystem des Patienten eingeleitet; beispielsweise führt bei der vvECMO ein in der Jugularvene oder der oberen Hohlvene liegender Katheter sauerstoffreiches Blut zum rechten Vorhof. Die extrakorporale Blutbehandlungsvorrichtung weist mindestens eine zwischen dem zuführenden und dem abführenden Schenkel angeordnete Pumpe zur Bewegung des Bluts des Patienten innerhalb der extrakorporalen Blutbehandlungsvorrichtung auf, wobei die Pumpe das Blut über die entsprechende Behandlungseinheit befördert, z.B. die Dialysemembran oder den Decarboxylator/Oxygenator. Das System weist Temperaturbeeinflussungsmittel zum Hervorrufen einer initialen, lokalen Temperaturabweichung $T_I$ in der Nähe einer ersten Stelle des Gefäßsystems des Patienten auf. Durch die Temperaturbeeinflussungsmittel wird eine wandernde Temperaturänderung im Blutfluss des Patienten eingeführt. Beispielsweise können die Temperaturbeeinflussungsmittel die Temperatur in einer zentralen Vene des Gefäßsystems des Patienten verändern, wie z.B. in der Jugularvene oder in der Femoralvene. Das System weist ferner einen ersten Temperatursensor zum Messen der lokalen Temperatur des Bluts des Patienten an einer zweiten Stelle des Gefäßsystems des Patienten stromabwärts zur ersten Stelle auf, sowie einen zweiten Temperatursensor zum Messen der lokalen Temperatur des Bluts des Patienten im zuführenden Schenkel der extrakorporalen Blutbehandlungsvorrichtung. Bevorzugt erfasst der erste Temperatursensor die Temperatur des Bluts des Patienten im stromabwärts des venösen Gefäßsystems gelegenen, arteriellen Gefäßsystem (transpulmonale Thermodilution). Die vorgesehenen Sensoren können vorteilhaft wie bekannte, in Dilutionsmessverfahren eingesetzte Sensoren ausgeführt sein. Für die Temperaturmessung ist insbesondere ein Platinwiderstandssensor geeignet, daneben sind jedoch auch andere Thermowiderstände oder Thermoelemente zweckgemäß. Ein mit dem ersten und dem zweiten Temperatursensor verbundenes Computersystem ist dazu ausgelegt, die lokale Bluttemperatur, die an der zweiten Stelle des Gefäßsystems des Patienten und im zuführenden Schenkel der extrakorporalen Blutbehandlungsvorrichtung gemessen wird, jeweils als Funktion der Zeit aufzuzeichnen und entsprechend eine erste und eine zweite Thermodilutionskurve ($TDK_1$, $TDK_2$) zu ermitteln und auszuwerten. Die programmtechnische Einrichtung zur Durchführung von Auswerteschritten eines Thermodilution ist dabei per se aus dem Stand der Technik bekannt (z.B. aus der deutschen Offenlegungsschrift DE 4 214 402 A1). Das Computersystem ist weiterhin dazu ausgelegt, aus der zweiten Thermodilutionskurve eine Temperaturabweichung $T_{EKBV}$ zu bestimmen, welche der extrakorporalen Blutbehandlungsvorrichtung zuzuordnen ist, und $T_I$ und $T_{EKBV}$ zur Bestimmung des kardiovaskulären Parameters in Beziehung zu setzen. Nach der Bestimmung des kardiovaskulären Parameters, beispielsweise des Herzzeitvolumens, kann die Flussrate der extrakorporalen Blutbehandlungsvorrichtung entsprechend gesteigert oder verringert werden, um die Pumpleistung der Herz-Kreislauf Situation des Patienten anzupassen. Beispielsweise kann die Flussrate der extrakorporalen Blutbehandlungsvorrichtung aber auch nur kurzfristig stark verringert bzw. auf null gesetzt werden, um eine Messung ohne Indikatorverlust in den extrakorporalen Kreislauf durchführen zu können.

Das erfindungsgemäße System ermöglicht es vorteilhaft, einen durch den extrakorporalen Kreislauf möglicherweise hervorgerufenen Indikatorverlust effektiv zu erfassen und nachfolgend zu korrigieren, so dass Fehler bei der Bestimmung kardiovaskulärer Parameter, wie z.B. des HZV, während einer extrakorporalen Blutbehandlung, z. B. während einer ECMO Behandlung, minimiert werden und damit die Genauigkeit und Zuverlässigkeit der Bestimmung erhöht wird. Das erfindungsgemäße System kann dabei unabhängig von der Anordnung/Flussrichtung der jeweiligen extrakorporalen Blutbehandlungsvorrichtung verwendet werden und ist somit vielseitig einsetzbar. Der Patient wird besser in die extrakorporale Blutbehandlungsvorrichtung integriert und deren Konfiguration optimal an den kardiovaskulären Zustand des Patienten angepasst.

In einer weiteren Ausführungsform des erfindungsgemäßen Systems kann die erste Stelle des Gefäßsystems des Patienten, in deren Nähe die Temperaturbeeinflussungsmittel die initiale, lokale Temperaturabweichung $T_I$ hervorrufen, stromaufwärts des zuführenden Schenkels der extrakorporalen Blutbehandlungsvorrichtung liegen. Der Begriff "stromaufwärts" bezieht sich dabei auf die Flussrichtung im zuführenden Schenkel der Blutbehandlungsvorrichtung. Beispielsweise kann der zuführende Schenkel der Blutbehandlungsvorrichtung in der unteren Hohlvene liegen, vorzugsweise unterhalb der Einmündung der Lebervene, während die Temperaturabweichung in der Jugularvene hervorgerufen wird. Die Anordnung führt zu einer geringstmöglichen Interferenz des erfindungsgemäßen Systems mit der extrakorporalen Blutbehandlungsvorrichtung, wodurch z.B. die Anzahl der erforderlichen Messzyklen vorteilhaft gering gehalten wird.

In einer bevorzugten Weiterbildung des erfindungsgemäßen Systems kann die extrakorporale Blutbehandlungsvorrichtung eine Blutbehandlungsvorrichtung mit einer Pumpe sein, die eine Flussrate von > 100 ml/min bereitstellt, besonders bevorzugt kann die Pumpe eine Flussrate von >200 ml/min und insbesondere bevorzugt eine Flussrate von > 300 ml/min bereitstellen. Bevorzugt kann es sich bei der extrakorporalen Blutbehandlungsvorrichtung eine Vorrichtung zur Decarboxylierung oder zur Membranoxygenierung (ECMO) handeln. Insbesondere bei extrakorporalen Blutbehandlungsvorrichtungen mit hoher Flussrate kann mittels des erfindungsgemäßen Systems die Genauigkeit der Bestimmung von kardiovaskulären Parametern erhöht werden, da der durch diese Vorrichtungen bedingte, mögliche Verlust des Indikators (z.B. der wandernden Temperaturänderung oder des Farbstoffs) in die entsprechende Vorrichtung akkurat und effektiv erfasst und nachfolgend korrigiert werden kann.

In einer weiteren Ausführungsform des erfindungsgemäßen Systems kann das Computersystem dazu ausgelegt sein, aus der Beziehung von $T_{EKBV}$ und $T_I$ den Anteil $A_{reEKBV}$ der wandernden Temperaturänderung zu bestimmen, welcher durch die extrakorporale Blutbehandlungsvorrichtung rezirkuliert. Beispielsweise kann ein Indikator (z. B. eine wandernde Temperaturänderung oder ein Farbstoff-Indikator) bei erhöhter Flussrate der Blutbehandlungsvorrichtung von der ersten Stelle des Gefäßsystems des Patienten größtenteils direkt in den zuführenden Schenkel ("Drainage-Katheder") der Vorrichtung abgezweigt werden. Durch ins-Verhältnissetzen der der extrakorporalen Blutbehandlungsvorrichtung zuzuordnenden Temperaturabweichung $T_{EKBV}$ mit $T_I$ kann der Anteil $A_{reEKBV}$ der in der Blutbehandlungsvorrichtung re-zirkulierenden, wandernden Temperaturänderung in Prozent bestimmt werden. Der Wert $A_{reEKBV}$ kann vorteilhaft zur Korrektur des zu bestimmenden kardiovaskulären Parameters herangezogen werden; alternativ kann der Wert dazu dienen, Systemparameter, beispielsweise die Flussrate der extrakorporalen Blutbehandlungsvorrichtung, für eine erneute Messung oder den kontinuierlichen Betrieb anzupassen.

In einer bevorzugten Implementierung des erfindungsgemäßen Systems können die Temperaturbeeinflussungsmittel manuelle oder automatisierte Injektionsmittel zum Injizieren einer Flüssigkeit sein, wobei sich eine Temperatur der Flüssigkeit von der Temperatur des Bluts des Patienten unterscheidet. Die Temperaturbeeinflussungsmittel können insbesondere geeignet sein, einen entsprechend temperierten Flüssigkeits-Bolus zu injizieren. Vorzugsweise werden Temperatur, Zeitpunkt und besonders bevorzugt auch Dauer der Bolus-Injektion durch einen weiteren, dritten Temperatursensor, der beispielsweise innerhalb oder in unmittelbarer Nähe der Temperaturbeeinflussungsmittel angeordnet ist, gemessen und dem Computersystem über entsprechende Verbindungsmittel zugeführt.

In einer bevorzugten Weiterbildung des erfindungsgemäßen Systems kann die erste Thermodilutionskurve mittels einer transpulmonalen Thermodilution ermittelt werden. Bei der aus dem Stand der Technik bekannten transpulmonalen Thermodilutionsmessung erfolgt zur Bestimmung der Thermodilutionskurve eine Injektion eines Thermoindikator-Bolus in die obere Hohlvene (V. cava sup.) eines Patienten und eine Messung der Temperaturantwort an einer Stelle im Körperkreislauf des Patienten, z.B. in der Femoralarterie eines Patienten. Vorteilhaft können aus der erhaltenen Thermodilutionskurve eine Vielzahl vaskulärer Parameter bestimmt werden, beispielsweise das Herzzeitvolumen HZV, das globale enddiastolische Volumen (GEDV), das intrathorakale Blutvolumen (ITBV) usw., sowie eine Reihe anderer Parameter und Indices abgeleitet werden, z. B. wird zusätzlich das extravasale Lungenwasser EVLW, ein Parameter für drohendes Lungenödem, errechnet. Zudem kann die Kontraktilität des Herzmuskels beurteilt werden ($dP_{max}$, GEF, CPI). Insbesondere kann es sich bei dem einen kardiovaskulären Parameter um das Herzzeitvolumen (HZV), das extravaskuläre Lungenwasser (EVLW) oder das globale enddiastolische Volumen (GEDV) handeln. Diese können in bekannter Art und Weise aus der arteriellen Thermodilutionskurve TDK berechnet werden; insbesondere können das Herzzeitvolumen $HZV_{TDart}$, die Erscheinungszeit AT, die mittlere Durchgangszeit $MTD_{TDart}$ und die exponentielle Abfallzeit $DST_{TDart}$ berechnet werden.

In einer weiteren Ausführungsform des erfindungsgemäßen Systems kann das Computersystem dazu eingerichtet sein, $T_I$ und $T_{EKBV}$ und die Fläche unter der ersten Thermodilutionskurve ($TDK_1$) zur Berechnung des Herzzeitvolumens in Beziehung zu setzen. Insbesondere ist beim erfindungsgemäßen System die Flussrate der Blutbehandlungsvorrichtung bekannt, während das Herzzeitvolumen und die in nicht die Blutbehandlungsvorrichtung abgezweigte Temperaturänderung unbekannt sind. Unter Zuhilfenahme der der Beziehung der Flussrate zum Quotienten der Temperaturabweichung im Gefäßsystem des Patienten ($T_I$-$T_{EKBV}$) und der Fläche unter der ersten Thermodilutionskurve kann das Herzzeitvolumen berechnet werden. Aus dieser Größe und aus den aus der TDK, ableitbaren Größen (MTT, DST) können z.B. die GEDV und andere kardiovaskuläre Parameter berechnet werden.

In einer bevorzugten Implementierung des erfindungsgemäßen Systems kann das Computersystem außerdem dazu ausgelegt sein, mit der Pumpe der extrakorporalen Blutbehandlungsvorrichtung zusammenzuwirken, derart, dass bei der Bestimmung des einen oder der mehreren kardiovaskulären Parameter/s $A_{reEKBV}$ < 30%. Dies kann beispielsweise erreicht werden, indem das Computersystem die Pumpe ansteuert und die Pumpenleistung entsprechend verringert oder zeitlich begrenzt ausgesetzt wird. Bevorzugt ist es, dass bei der Bestimmung des einen oder der mehreren kardiovaskulären Parameter/s $A_{reEKBV} < 20\%$, insbesondere bevorzugt ist $A_{reEKBV} < 10\%$.

In einer besonderen Weiterbildung des erfindungsgemäßen Systems können sich die erste Stelle des Gefäßsystems eines Patienten, in deren Nähe die Temperaturbeeinflussungsmittel eine initiale lokale Temperaturabweichung hervorrufen, und der zuführende Schenkel der extrakorporalen Blutbehandlungsvorrichtung kranial befinden bezüglich einer Ebene, in der die Herzklappen des Patienten liegen. Die Anordnung ist vorteilhaft, da für die spezifische Messanordnung im Hinblick auf die Berechnung einer Vielzahl kardiovaskulärer Parameter bereits empirisch zu berücksichtigende Konstanten vorliegen. Beispielsweise sind die in die Steward-Hamilton Gleichung einzufügenden

$$HZV = \frac{V_L(T_B - T_L)K_1 K_2}{\int \Delta T_B(t)dt}$$

Konstanten $K_1$ und $K_2$ für die entsprechend durchgeführte transpulmonale Thermodilution bekannt, d.h. für eine Anordnung, in der der Temperatur-Bolus über die Jugularvene in die obere Hohlvene injiziert und in der Femoralarterie eines Patienten erfasst wird.

Nachfolgend werden anhand der zugehörigen Zeichnung Beispiele bevorzugte Ausführungsformen der vorliegenden Erfindung näher erläutert.

Die Zeichnung ist dabei rein schematische und, aus Gründen der Anschaulichkeit, nicht maßstabsgetreue Darstellung. Insbesondere können Verhältnisse zwischen den Abmessungen, vor allem Durchmesser, Schlauchlängen und Außenabmessungen von tatsächlichen Ausführungen abweichen. In der Praxis können die Abmessungen anhand der Anforderungen im Einzelfall sowie anhand gängiger Standardteile dimensioniert werden.

**Kurze Beschreibung der Figur**

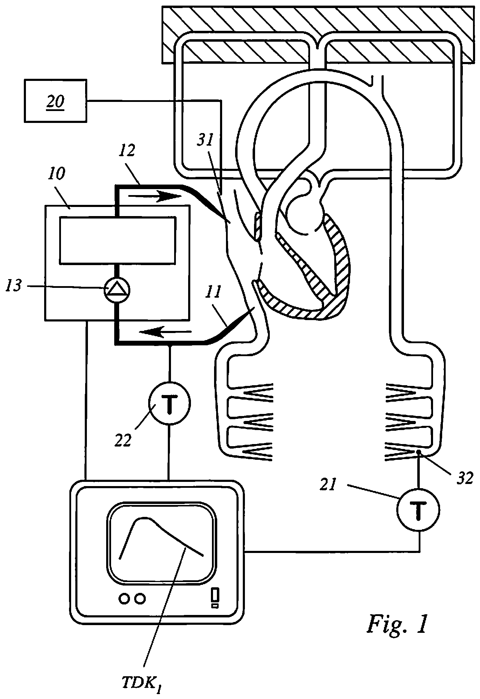

Fig. 1 zeigt eine schematische Übersicht der erfindungsgemäßen Messanordnung in einer bevorzugten Ausführungsform und des Gefäßsystems eines Patienten.

**Bevorzugte Ausführung der Erfindung**

Fig. 1 zeigt eine schematische Übersicht der erfindungsgemäßen Messanordnung und des Gefäßsystems eines Patienten, wobei die Vorrichtung zur extrakorporalen Blutbehandlung (10) mit dem venösen Gefäßsystem des Patienten über einen zuführenden Schenkel (11; "Drainage") und einen abführenden Schenkel (12) verbunden ist. Bei der extrakorporalen Blutbehandlungsvorrichtung kann es sich beispielsweise um eine Vorrichtung zur extrakorporalen Membranoxygenierung (vvECMO, wie in Fig. 1 dargestellt) oder um eine Vorrichtung zur Leberdialyse handeln. Der zuführende Schenkel (11) ist beispielsweise über die Femoralvene eingeführt und kommt in der unteren Hohlvene (V. cava inferior) unterhalb der Einmündung der Lebervene zu liegen. Der abführende Schenkel (12) ist beispielsweise über die Jugularvene eingeführt und kommt in der oberen Hohlvene (V. cava superior) unmittelbar vor dem rechten Vorhof zu liegen. Über den zuführenden Schenkel (11) wird der Blutbehandlungsvorrichtung im Falle der ECMO sauerstoffarmes, venöses Blut zugeführt, während das oxygenierte Blut über den abführenden Schenkel (12) zum rechten Vorhof gelangt. Das erfindungsgemäße System kann ebenfalls in Situationen angewendet werden, in denen eine vaECMO verwendet wird, bei welcher das deoxygenierte Blut über die Femoralvene zur ECMO geleitet und das oxygenierte Blut über die Femoralarterie in das Gefäßsystem des Patienten zurückgeleitet wird. In dieser Anordnung übernimmt die ECMO die gesamte Pumpleistung.

Der kardiovaskuläre Parameter wird mittels Thermodilution bestimmt; mittels der Thermodilution lassen sich auch mehrere kardiovaskulärer Parameter bestimmen und/oder errechnen. Bei den erfindungsgemäßen Temperaturbeeinflussungsmitteln (20) handelt es sich vorzugsweise um eine Injektionsvorrichtung, mittels derer eine gekühlte Flüssigkeit als Bolus in das Gefäßsystem des Patienten injiziert wird. Prinzipiell kann die Temperaturänderung an einer beliebigen Stelle des zentralen, venösen Gefäßsystems des Patienten erzeugt werden. Vorzugsweise liegen die abführende Schenkel der ECMO (über welchen dem Gefäßsystem des Patienten oxygeniertes Blut zugeführt wird) und die erste Stelle (31) bezüglich der Herzklappenebene kranial. Besonders bevorzugt wird, wie in Fig. 1 gezeigt, an einer ersten Stelle (31), z. B. der Jugularvene, stromaufwärts des zuführenden Schenkels (11) der ECMO eine Bolus-Injektion zur Thermodilutionsmessung vorgenommen. Eine in der Jugularvene erzeugte wandernde Temperaturänderung gelangt über das rechte Herz und die pulmonale Zirkulation in das linke Herz und von dort über die Aorta in die systemische Zirkulation. Die durch die Bolus-Injektion erzeugte initiale Temperaturabweichung $T_I$ wird dabei z.B. über einen entsprechenden Algorithmus berechnet oder gemessen. Vorzugsweise können die Temperaturbeeinflussungsmittel Temperatur, Zeitpunkt und gegebenenfalls auch Dauer der Bolus-Injektion erfassen, beispielsweise mittels eines Temperatursensors, welcher innerhalb oder in unmittelbarer Nähe der Temperaturbeeinflussungsmittel angeordnet ist. Die Temperaturbeeinflussungsmittel können bevorzugt die Messergebnisse einem Computersystem (40) zuführen, welches mit dem erfindungsgemäßen System über Verbindungsmittel verbunden ist.

An einer zweiten Stelle (32) des Gefäßsystems des Patienten stromabwärts zur ersten Stelle (31), ist der erste Temperatursensor (21) zum Messen der lokalen Temperatur des Bluts des Patienten angeordnet, hier z. B. in der Femoralarterie. Ein weiterer Temperatursensor (22) ist im zuführenden Schenkel (11) der extrakorporalen Blutbehandlungsvorrichtung angeordnet. In Abhängigkeit von der Flussrate der extrakorporalen Blutbehandlungsvorrichtung, z.B. bei hoher Flussrate >500ml/min, oder in Abhängigkeit von der Lagebeziehung Injektionsort/zuführen der Schenkel wird ein Teil des injizierten Temperatur-Bolus in den extrakorporalen Kreislauf abgezweigt. Dieser Teil des injizierten TemperaturBolus wird durch den zweiten Temperatursensor (22) erfasst. Das mit dem ersten (21) und dem zweiten Temperatursensor (22) über Verbindungsmittel verbundene Computersystem (40) zeichnet jeweils die erfasste Temperatur als Funktion der Zeit auf und ermittelt entsprechend eine erste und eine zweite Thermodilutionskurve ($TDK_1$, $TDK_2$). Die programmtechnische Einrichtung zur Durchführung von Auswerteschritten eines Thermodilution ist dabei perse aus dem Stand der Technik bekannt (z.B. aus der deutschen Offenlegungsschrift DE 4 214 402 A1). Das Computersystem ist weiterhin dazu ausgelegt, aus der zweiten Thermodilutionskurve $TDK_2$ eine Temperaturabweichung $T_{EKBV}$ zu bestimmen, welche der extrakorporalen Blutbehandlungsvorrichtung zuzuordnen ist, und $T_I$ und $T_{EKBV}$ zur Bestimmung des kardiovaskulären Parameters in Beziehung zu setzen. Das erfindungsgemäße System ermöglicht es vorteilhaft, einen durch den extrakorporalen Kreislauf möglicherweise hervorgerufenen Indikatorverlust effektiv zu erfassen und nachfolgend zu korrigieren, so dass Fehler bei der Bestimmung kardiovaskulärer Parameter, wie z.B. des HZV, während einer extrakorporalen Blutbehandlung, z. B. während einer ECMO Behandlung, minimiert werden und damit die Genauigkeit und Zuverlässigkeit der Bestimmung erhöht wird. Das erfindungsgemäße System kann dabei unabhängig von der Anordnung/Flussrichtung der jeweiligen extrakorporalen Blutbehandlungsvorrichtung verwendet werden und ist somit vielseitig einsetzbar. Der Patient wird besser in die extrakorporale Blutbehandlungsvorrichtung integriert und deren Konfiguration optimal an den kardiovaskulären Zustand des Patienten angepasst.

In einer weiteren Ausführungsform des erfindungsgemäßen Systems kann die erste Stelle des Gefäßsystems des Patienten, in deren Nähe die Temperaturbeeinflussungsmittel die initiale, lokale Temperaturabweichung $T_I$ hervorrufen, in der Nähe einer ersten Stelle des Gefäßsystems des Patienten stromaufwärts des zuführenden Schenkels (11) der extrakorporalen Blutbehandlungsvorrichtung liegen. Der Begriff "stromaufwärts" bezieht sich dabei auf die Flussrichtung im zuführenden Schenkel der Blutbehandlungsvorrichtung. Beispielsweise kann der zuführende Schenkel der Blutbehandlungsvorrichtung in der unteren Hohlvene liegen, während die Temperaturabweichung in der Jugularvene hervorgerufen wird. Die Anordnung führt zu einer geringstmöglichen Interferenz des erfindungsgemäßen Systems mit der extrakorporalen Blutbehandlungsvorrichtung, wodurch z.B. die Anzahl der erforderlichen Messzyklen vorteilhaft gering gehalten wird.

**Patentansprüche**

1. System zum Bestimmen eines kardiovaskulären Parameters eines Patienten (30), welches dazu eingerichtet ist, mit einer Vorrichtung zur extrakorporalen Blutbehandlung (10) zusammenzuwirken, welche mit dem venösen Gefäßsystem des Patienten über einen zuführenden (11) und einen abführenden Schenkel (12) verbunden ist, wobei die extrakorporale Blutbehandlungsvorrichtung (10) mindestens eine zwischen dem zuführenden (11) und dem abführenden Schenkel (12) angeordnete Pumpe (13) zur Bewegung des Bluts des Patienten innerhalb der extrakorporalen Blutbehandlungsvorrichtung aufweist,
wobei das System folgendes aufweist:

   a. Temperaturbeeinflussungsmittel (20) zum Hervorrufen einer initialen lokalen Temperaturabweichung $T_I$ in der Nähe einer ersten Stelle (31) des Gefäßsystems des Patienten, wodurch eine wandernde Temperaturänderung im Blutfluss des Patienten eingeführt wird,
   b. einen ersten Temperatursensor (21) zum Messen der lokalen Temperatur des Bluts des Patienten an einer zweiten Stelle (32) des Gefäßsystems des Patienten stromabwärts zur ersten Stelle (31),
   c. einen zweiten Temperatursensor (22) zum Messen der lokalen Temperatur des Bluts des Patienten im zuführenden Schenkel (11) der extrakorporalen Blutbehandlungsvorrichtung,
   d. ein Computersystem (40), welches mit dem ersten (21) und dem zweiten Temperatursensor (22) verbunden ist, und dazu ausgelegt ist, die lokale Bluttemperatur, die an der zweiten Stelle (32) des Gefäßsystems des Patienten und im zuführenden Schenkel (11) der extrakorporalen Blutbehandlungsvorrichtung gemessen wird, jeweils als Funktion der Zeit aufzuzeichnen und entsprechend eine erste und eine zweite Thermodilutionskurve ($TDK_1$, $TDK_2$) zu ermitteln und auszuwerten, und weiterhin dazu ausgelegt ist, aus der zweiten Thermodilutionskurve eine Temperaturabweichung $T_{EKBV}$ zu bestimmen, welche der extrakorporalen Blutbehandlungsvorrichtung zuzuordnen ist, und $T_I$ und $T_{EKBV}$ zur Bestimmung des kardiovaskulären Parameters in Beziehung zu setzen.

2. System gemäß Anspruch 1, wobei die erste Stelle des Gefäßsystems des Patienten stromaufwärts des zuführenden Schenkels (11) der extrakorporalen Blutbehandlungsvorrichtung liegt.

3. System gemäß einem der Ansprüche 1 oder 2, wobei die extrakorporale Blutbehandlungsvorrichtung eine Blutbehandlungsvorrichtung mit einer Pumpe ist, die eine Flussrate von > 100 ml/min bereitstellt.

4. System gemäß einem der vorhergehenden Ansprüche, wobei das Computersystem dazu ausgelegt ist, aus der Beziehung von $T_{EKBV}$ und $T_I$ einen Anteil $A_{reEKBV}$ der wandernden Temperaturänderung zu bestimmen, welcher durch die extrakorporale Blutbehandlungsvorrichtung rezirkuliert.

5. System gemäß einem der vorhergehenden Ansprüche, wobei die Temperaturbeeinflussungsmittel Injektionsmittel zum Injizieren einer Flüssigkeit aufweisen, wobei sich eine Temperatur der Flüssigkeit von der Temperatur des Bluts des Patienten unterscheidet.

6. System gemäß einem der vorhergehenden Ansprüche, welches dazu ausgelegt ist, die erste Thermodilutionskurve mittels einer transpulmonalen Thermodilution zu ermitteln.

7. System gemäß einem der vorhergehenden Ansprüche, wobei der kardiovaskuläre Parameter das Herzzeitvolumen (HZV), das extravaskuläre Lungenwasser (EVLW) oder das globale enddiastolische Volumen (GEDV) ist.

8. System gemäß einem der vorhergehenden Ansprüche 6 oder 7, wobei das Computersystem dazu eingerichtet ist, $T_I$ und $T_{EKBV}$ und die Fläche unter der ersten Thermodilutionskurve zur Berechnung des Herzzeitvolumens in Beziehung zu setzen.

9. System gemäß einem der Ansprüche 4 bis 8, wobei das Computersystem außerdem dazu ausgelegt ist, mit der Pumpe der extrakorporalen Blutbehandlungsvorrichtung zusammenzuwirken, derart, dass bei der Bestimmung des einen oder der mehreren kardiovaskulären Parameters $A_{EKBV} < 30\%$.

10. System gemäß einem der vorhergehenden Ansprüche, wobei die erste Stelle des Gefäßsystems eines Patienten, in deren Nähe eine initiale lokale Temperaturabweichung hervorzurufen die Temperaturbeeinflussungsmittel ausgelegt sind, und der abführende Schenkel der extrakorporalen Blutbehandlungsvorrichtung sich kranial befinden bezüglich einer Ebene, in der die Herzklappen des Patienten liegen.

**Claims**

1. System for determining a cardiovascular parameter of a patient (30) which is configured to interact with an apparatus for extracorporeal blood treatment (10) which is connected to the venous vascular system of the patient via an inflow line (11) and an outflow line (12), wherein the extracorporeal blood treatment apparatus (10) comprises at least one pump (13) arranged between the inflow line (11) and the outflow line (12) for moving the blood of the patient within the extracorporeal blood treatment apparatus, said system comprising the following:

    a. temperature influencing means (20) for causing an initial local temperature deviation $T_1$ in the vicinity of a first point (31) of the vascular system of the patient, so that a traveling temperature change is introduced in the blood flow of the patient;
    b. a first temperature sensor (21) for measuring the local temperature of the blood of the patient at a second point (32) of the vascular system of the patient downstream of the first point (31);
    c. a second temperature sensor (22) for measuring the local temperature of the blood of the patient in the inflow line (11) of the extracorporeal blood treatment apparatus,
    d. a computer system (40) which is connected to the first temperature sensor (21) and the second temperature sensor (22) and is configured to record the local blood temperature, which is measured at the second point (32) of the vascular system of the patient and in the inflow line (11) of the extracorporeal blood treatment apparatus, respectively, as a function of time, and to determine and evaluate accordingly a first and a second thermodilution curve ($TDK_1$, $TDK_2$), and is further configured to determine, from the second thermodilution curve, a temperature deviation $T_{EKBV}$ which is to be allocated to the extracorporeal blood treatment apparatus, and to correlate $T_1$ and $T_{EKBV}$ to determine the cardiovascular parameter.

2. System according to claim 1, wherein the first point of the vascular system of the patient is located upstream of the inflow line (11) of the extracorporeal blood treatment apparatus.

3. System according to one of claims 1 or 2, wherein the extracorporeal blood treatment apparatus is a blood treatment apparatus with a pump providing a flow rate of > 100 mL/min.

4. System according to one of the preceding claims, wherein the computer system is configured to determine, from the relation of $T_{EKBV}$ and $T_1$, a proportion $A_{reEKBV}$ of the traveling temperature change recirculating through the extracorporeal blood treatment apparatus.

**5.** System according to one of the preceding claims, wherein the temperature influencing means comprise injection means for injecting a liquid, a temperature of the liquid differing from the temperature of the blood of the patient.

**6.** System according to one of the preceding claims, which is adapted to determine the first thermodilution curve by means of transpulmonary thermodilution.

**7.** System according to one of the preceding claims, wherein the cardiovascular parameter is cardiac output (CO), extra vascular lung water (EVLW), or global end-diastolic volume (GEDV).

**8.** System according to one of the preceding claims 6 or 7, wherein the computer system is configured to correlate $T_1$ and $T_{EKBV}$ and the area under the first thermodilution curve for calculating cardiac output.

**9.** System according to one of the preceding claims 4 to 8, wherein the computer system is also configured to interact with the pump of the extracorporeal blood treatment apparatus such that when determining the one or more cardiovascular parameters $A_{EKBV} < 30\%$.

**10.** System according to one of the preceding claims, wherein the first point of the vascular system of a patient, in the vicinity of which point the temperature influencing means are adapted to cause an initial local temperature deviation, and the outflow line of the extracorporeal blood treatment apparatus are located cranially with respect to a plane in which the heart valves of the patient are located.

## Revendications

**1.** Système pour la détermination d'un paramètre cardiovasculaire d'un patient (30), qui est conçu pour interagir avec un dispositif de traitement de sang extracorporel (10) qui est relié avec le système veineux du patient par l'intermédiaire d'une branche d'alimentation (11) et d'une branche d'évacuation (12), dans lequel le dispositif de traitement de sang extracorporel (10) comprend au moins une pompe (13), disposée entre les branches d'alimentation (11) et d'évacuation (12), pour le déplacement du sang du patient à l'intérieur du dispositif de traitement de sang extracorporel,
dans lequel le système comprend ce qui suit :

a. des moyens de contrôle de la température (20) afin de produire un écart de température local initial $T_I$ à proximité d'un premier point (31) du système vasculaire du patient, ce qui introduit un changement de température itinérant dans le flux sanguin du patient,

b. un premier capteur de température (21) pour la mesure de la température locale du sang du patient au niveau d'un deuxième point (32) du système vasculaire du patient en aval du premier point (31),

c. un deuxième capteur de température (22) pour la mesure de la température locale du sang du patient dans la branche d'alimentation (11) du dispositif de traitement de sang extracorporel,

d. un système informatique (40) qui est relié avec les premier (21) et deuxième capteurs de température (22) et qui est conçu pour enregistrer la température locale du sang, qui est mesurée au niveau du deuxième point (32) du système vasculaire du patient et dans la branche d'alarme (11) du dispositif de traitement de sang extracorporel, respectivement en fonction du temps et pour déterminer et analyser en conséquence des première et deuxième courbe de thermodilution ($TDK_1$, $TDK_2$) et qui est conçu en outre pour déterminer, à partir de la deuxième courbe de thermodilution, un écart de température $T_{EKBV}$ qui peut être attribué au dispositif de traitement de sang extracorporel et pour mettre en relation $T_I$ et $T_{EKBV}$ pour la détermination du paramètre cardiovasculaire.

**2.** Système selon la revendication 1, dans lequel le premier point du système vasculaire du patient se trouve en amont de la branche d'alimentation (11) du dispositif de traitement de sang extracorporel.

**3.** Système selon l'une des revendications 1 ou 2, dans lequel le dispositif de traitement de sang extracorporel est un dispositif de traitement de sang extracorporel avec une pompe qui génère un débit > 100 ml/min.

**4.** Système selon l'une des revendications précédentes, dans lequel le système informatique est conçu pour déterminer, à partir de la relation entre $T_{EKBVC}$ et $T_I$, une part $A_{reEKBV}$ du changement de température itinérant qui est remis en circulation par le dispositif de traitement de sang extracorporel.

**5.** Système selon l'une des revendications précédentes, dans lequel les moyens de contrôle de la température comprennent des moyens d'injection pour l'injection d'un liquide, dans lequel une température du liquide est différente de la température du sang du patient.

**6.** Système selon l'une des revendications précédentes, qui est conçu pour déterminer la première courbe de thermodilution au moyen d'une thermodilution transpulmonaire.

7. Système selon l'une des revendications précédentes, dans lequel le paramètre cardiovasculaire est le débit cardiaque (HZV), l'eau pulmonaire extravasculaire (EVLW) ou le volume télédiastolique global (GEDV).

8. Système selon l'une des revendications 6 ou 7, dans lequel le système informatique est conçu pour mettre en relation $T_I$ et $T_{EKBV}$ et la surface sous la première courbe de thermodilution afin de calculer le débit cardiaque.

9. Système selon l'une des revendications 4 à 8, dans lequel le système informatique est conçu en outre pour interagir avec la pompe du dispositif de traitement de sang extracorporel de sorte que, lors de la détermination du ou des paramètres cardiovasculaires, $A_{EKBV} < 30\ \%$.

10. Système selon l'une des revendications précédentes, dans lequel le premier point du système vasculaire d'un patient, à proximité duquel les moyens de contrôle de la température sont conçus pour générer un écart de température local initial et la branche d'évacuation du dispositif de traitement de sang extracorporel se trouvent en position crâniale par rapport à un plan dans lequel se trouvent les valvules cardiaques du patient.

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2011004143 A1

- DE 4214402 A1